(19) Europäisches Patentamt
European Patent Office
Office européen des brevets

(11) EP 1 042 252 B1

(12) # FASCICULE DE BREVET EUROPEEN

(45) Date de publication et mention de la délivrance du brevet:
**26.03.2003 Bulletin 2003/13**

(21) Numéro de dépôt: **98963593.3**

(22) Date de dépôt: **22.12.1998**

(51) Int Cl.$^7$: **C04B 35/447**, A61L 27/00, A61K 6/033, C04B 35/64

(86) Numéro de dépôt international:
**PCT/FR98/02827**

(87) Numéro de publication internationale:
**WO 99/033766 (08.07.1999 Gazette 1999/27)**

(54) **PROCEDE DE FABRICATION D'UNE CERAMIQUE APATITIQUE, EN PARTICULIER POUR USAGE BIOLOGIQUE**

VERFAHREN ZUR HERSTELLUNG VON APATITKERAMIK, INSBESONDERE ZUR BIOLOGISCHEN VERWENDUNG

METHOD FOR MAKING APATITE CERAMICS, IN PARTICULAR FOR BIOLOGICAL USE

(84) Etats contractants désignés:
**BE CH DE ES GB IT LI NL SE**

(30) Priorité: **23.12.1997 FR 9716357**

(43) Date de publication de la demande:
**11.10.2000 Bulletin 2000/41**

(73) Titulaire: **COMMISSARIAT A L'ENERGIE ATOMIQUE**
**75015 Paris (FR)**

(72) Inventeurs:
- **CARPENA, Joelle**
  **Le Turquet, F-13490 Jouques (FR)**
- **DONAZZON, Benoît**
  **F-31570 Lanta (FR)**
- **LACOUT, Jean-Louis**
  **F-31000 Toulouse (FR)**
- **FRECHE, Michèle**
  **F-31130 Fonsegrives (FR)**

(74) Mandataire: **Audier, Philippe André et al**
**Brevalex,**
**3, rue du Docteur Lancereaux**
**75008 Paris (FR)**

(56) Documents cités:
**EP-A- 0 548 365**

- **YAMASAKI N ET AL: "POROUS HYDROXYAPATITE CERAMICS PREPARED BY HYDROTHERMAL HOT-PRESSING" JOURNAL OF MATERIALS SCIENCE LETTERS, vol. 9, no. 10, 1 octobre 1990, pages 1150-1151, XP000201972**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

### Domaine technique

La présente invention a pour objet un procédé de fabrication d'une céramique apatitique, utilisable en particulier pour la réalisation d'apatites à usage biologique.
Les apatites sont des matériaux qui présentent un grand intérêt dans divers domaines, par exemple en agriculture comme engrais, en orthopédie en tant que biomatériaux et en chimie analytique comme support de chromatographie.
Les apatites sont des composés de formule générale :

$$Me_{10}(XO_4)_6Y_2 \qquad (I)$$

dans laquelle Me représente un ou plusieurs cations, $XO_4$ représente $PO_4$ et/ou d'autres groupements anioniques, et Y représente un ou plusieurs anions tels que OH, Cl et F. Parmi ces apatites, l'hydroxyapatite phosphocalcique :

$$Ca_{10}(PO_4)_6(OH)_2 \qquad (II)$$

est le composé le plus connu.
Les apatites de formule (I) peuvent admettre diverses substitutions, aussi bien dans les sites cationiques (Me) que dans les sites anioniques ($XO_4$ et/ou $Y_2$).
Selon les diverses utilisations envisagées, on peut utiliser ces possibilités de substitution pour améliorer les propriétés physiques et/ou chimique de ces apatites.
Par ailleurs, les apatites, en particulier celles de formule (II), possèdent la possibilité d'être non-stoechiométrique, c'est-à-dire de présenter un rapport atomique calcium/phosphore différent de celui de l'apatite stoechiométrique de formule (II), qui est de 1,677.
En fait, dans les apatites non-stoechiométriques, ce rapport est généralement inférieur à 1,677. Cette non-stoechiométrie s'explique en particulier par la présence de lacunes dans les sites cationiques/calcium, et/ou par la présence d'ions $HPO_4^{2-}$ en substitution des ions phosphate. Une formule générale des apatites peut être donnée par :

$$Ca_{10-x}V_x(PO_4)_{6-y}(HPO_4)_y\,(OH)_{2+y-2x} \qquad (III)$$

où V représente une lacune et dans laquelle x et y sont tels que $x < 1$, $y < 1$ et $y \leq x$. Lorsque $x = y = 0$, l'apatite est stoechiométrique.
Parmi les apatites, on connaît des apatites biologiques non stoechiométriques qui forment la partie minérale des tissus durs, des dents et des os.
L'utilisation d'apatites dans les domaines biologique, orthopédique ou dentaire repose sur leur parfaite biocompatibilité. On attribue cette biocompatibilité à la structure et à la composition de l'hydroxyapatite qui sont très voisines de celles de la partie minérale des tissus calcifiés. La plupart des orthophosphates de calcium dont le phosphate tricalcique, le phosphate dicalcique et le phosphate octocalcique, possèdent également d'excellentes propriétés de biocompatibilité. Par ailleurs, on reconnaît aux hydroxyapatites, et plus généralement aux orthophosphates de calcium, des propriétés d'ostéoconduction.
En ce qui concerne leur solubilité, les hydroxyapatites sont considérées comme faiblement solubles en milieu biologique alors que les autres orthophosphates de calcium le sont beaucoup plus, comme on peut le voir dans le tableau annexé qui mentionne les solubilités de ces différents phosphates.
Les utilisations biologiques des apatites sont nombreuses. En effet, on peut les utiliser soit en comblement (sous forme de poudre ou de granulés), soit en recouvrement (sous forme de poudre projetée par projection plasma), soit encore sous forme de pièces massives destinées à des comblements résistants ou des fixations : coins d'ostéotomie, vis, cages interstomatiques, etc. Pour ces dernières utilisations, il est donc nécessaire de préparer l'apatite sous la forme d'une pièce massive.

### Etat de la technique antérieur

Jusqu'à présent la préparation de pièces massives en apatite a été effectuée à partir d'apatites pulvérulentes

que l'on soumet à un frittage à haute température (supérieure à 1 000°C) sous charge ou non.

Le document Bioceramics, Vol. 10, 1997, pages 75 à 78, illustre la densification d'hydroxyapatite polycristalline par compression à chaud à 1 165°C, sous 10 MPa.

Les procédés qui sont à l'heure actuelle utilisés pour la préparation de biomatériaux en hydroxyapatites, nécessitent donc la préparation préalable de la poudre d'apatite, sa mise en forme granulométrique et son frittage à une température élevée, selon divers procédés tels que frittage naturel, frittage sous charge et frittage après utilisation de barbotine.

Ces techniques permettent d'obtenir des pièces massives possédant de bonnes propriétés mécaniques, mais elles présentent l'inconvénient de nécessiter la mise en oeuvre de traitements thermiques à température élevée, ce qui conduit à :

- un coût énergétique élevé pour la préparation de l'apatite,
- une transformation partielle de l'hydroxyapatite en oxyapatite, et
- des difficultés pour introduire dans la pièce en apatite, des espèces volatiles ou dégradables à la température du traitement thermique.

L'article de Yamasaki et al. en Journal of Materials Science Letters, Vol. 9, No. 10, 1-10-1990, "Porous Hydroxyapatite Ceramics prepared by Hydrothermal hot-Pressing" décrit la préparation de pièces par voie hydrothermale.

## Exposé de l'invention

La présente invention a précisément pour objet un procédé de fabrication de céramiques apatitiques, qui présente l'avantage de conduire à des pièces ayant de bonnes propriétés mécaniques, sans qu'il soit nécessaire de mettre en oeuvre un traitement thermique à température élevée.

Selon l'invention, le procédé de fabrication d'une pièce massive en céramique apatitique, comprend les étapes suivantes :

a) préparer un mélange homogène de poudres comprenant au moins deux phosphates de calcium choisis parmi : $Ca(H_2PO_4)_2$, $Ca(H_2PO_4)_2.H_2O$, $Ca(HPO_4)$, $Ca(HPO_4).2H_2O$, $Ca_3(PO_4)_2$ de variété $\alpha$ ou $\beta$, et $Ca_4(PO_4)_2O$, en quantités telles que le mélange correspond à l'obtention d'une hydroxyapatite de formule :

$$Ca_{10}(PO_4)_6(OH)_2 \quad (II)$$

ayant un rapport atomique Ca/P égal à 1,667, ou d'une hydroxyapatite non-stoechiométrique de formule :

$$Ca_{10-x}V_x(PO_4)_{6-y}(HPO_4)_y\ (OH)_{2+y-2x} \quad (III)$$

où V représente une lacune et x et y sont tels que $x < 1$, $y < 1$ et $y \leq x$, ayant un rapport atomique Ca/P inférieur à 1,667.

b) compacter le mélange de poudres obtenu dans l'étape a), à la température ambiante, sous une pression de 100 à 500 MPa, pour obtenir une pièce compactée ; et

c) soumettre la pièce compactée à un traitement hydrothermal dans une enceinte étanche contenant un milieu aqueux, à une température de 100 à 500°C, pendant une durée d'au moins 8 heures.

Le procédé tel que décrit ci-dessus permet ainsi d'obtenir une hydroxyapatite stoechiométrique ou non-stoechiométrique, à basse température, puisque la température utilisée dans la dernière étape ne dépasse pas 500°C, ce qui présente de nombreux avantages en ce qui concerne le coût énergétique et la possibilité d'introduire dans l'hydroxyapatite des espèces volatiles ou instables à des températures supérieures à 500°C. Par ailleurs, les pièces massives obtenues ont de bonnes propriétés mécaniques et peuvent être facilement usinées.

On peut aussi utiliser le procédé de l'invention pour fabriquer des céramiques apatitiques, stoechiométriques de formule :

$$Ca_{10}(PO_4)_6(OH)_2 \quad (II)$$

ou non-stoechiométriques de formule :

$$Ca_{10-x}V_x(PO_4)_{6-y}(HPO_4)_y\ (OH)_{2+y-2x} \quad (III)$$

où V représente une lacune et x et y sont tels que $x < 1$, $y < 1$ et $y \leq x$,
dans lesquelles Ca, $PO_4$ et/ou OH sont remplacés en partie par d'autres métaux dans le cas de Ca, et/ou d'autres anions dans le cas de $PO_4$ et OH.

Selon ce mode de réalisation de l'invention, on prépare dans l'étape a), un mélange de poudres comprenant de plus au moins un composé choisi parmi les sels, oxydes et hydroxydes de métaux alcalins, de métaux alcalino-terreux, d'argent ou d'autres métaux, et l'oxyde de silicium, ledit mélange étant capable de former une apatite stoechiométrique de formule :

$$Ca_{10}(PO_4)_6(OH)_2 \quad (II)$$

ou une apatite non-stoechiométrique de formule :

$$Ca_{10-x}V_x(PO_4)_{6-y}(HPO_4)_y\ (OH)_{2+y-2x} \quad (III)$$

où V représente une lacune et x et y sont tels que $x < 1$, $y < 1$ et $y \leq x$,
dans lesquelles Ca, $PO_4$ et/ou OH sont remplacés respectivement en partie par d'autres métaux et/ou d'autres anions.

Pour la réalisation de ce mélange, les sels utilisés peuvent être choisis parmi les phosphates, les silicates, les citrates, les nitrates, les carbonates et les halogénures.

Ce mode de réalisation du procédé de l'invention permet d'inclure dans la structure apatitique des cations ou des anions présentant un intérêt particulier pour l'application envisagée.

Ainsi, dans le cas où le procédé est mis en oeuvre pour préparer une céramique apatitique à usage biologique, il peut être intéressant d'inclure dans cette structure du strontium en préparant un mélange de poudres comprenant du phosphate de strontium et de calcium de formule :

$$Ca_2Sr(PO_4)_2.$$

ou en remplaçant un ou plusieurs des composés calciques phosphatés utilisés par des composés strontiques phosphatées analogues, ou par des composés mixtes calcium-strontium. On pourra utiliser les composés : $Sr(H_2PO_4)_2$, $Sr(H_2PO_4)_2.H_2O$, $Sr(HPO_4)$, $Sr(PHO_4).2H_2O$, $Sr_3(PO_4)_2$ et $Sr_4(PO_4)_2O$.

En effet, la présence de strontium dans une céramique apatitique à usage biomédical est intéressante car elle facilite la repousse osseuse.

Pour des applications biologiques, on peut aussi ajouter au mélange de poudres du carbonate de calcium afin d'obtenir une apatite carbonatée similaire à l'os.

On peut encore ajouter au mélange un composé de silicium choisi parmi l'oxyde de silicium $SiO_2$, le métasilicate de calcium $CaSiO_3$, et les métasilicates d'autres métaux, en vue d'obtenir une céramique apatique silicatée, soit une hydroxyapatite dans laquelle les anions $PO_4$ sont substitués par des anions $SiO_4$. De telles apatites silicatées permettent la fixation de protéines.

Le procédé de l'invention permet donc d'obtenir divers matériaux adaptés à l'application envisagée.

Le procédé de l'invention repose sur une réaction hydrothermale entre les divers constituants du mélange de poudres, préalablement compactés.

Dans l'étape a) de ce procédé, on prépare un mélange de poudres capable de conduire à une hydroxyapatite de formule :

$$Ca_{10}(PO_4)_6(OH)_2$$

dans laquelle les anions et/ou les cations peuvent être éventuellement substitués par d'autres cations et anions.

Le mélange peut être préparé par broyage de ces constituants, à une granulométrie inférieure à 100 μm.

Les constituants du mélange sont choisis en fonction de la composition que l'on veut obtenir.

Ainsi, lorsqu'on veut préparer une céramique apatitique du type hydroxyapatite phosphocalcique, on utilise un mélange de divers composés de phosphates de calcium tels que le phosphate monocalcique $Ca(H_2PO_4)_2$, le phosphate monocalcique hydraté $Ca(H_2PO_4)_2,H_2O$, le phosphate bicalcique anhydre $Ca(HPO_4)$ ou dihydraté $Ca(HPO_4)$, $2H_2O$, le phosphate tricalcique de variété $\alpha$ ou $\beta$ $Ca_3(PO_4)_2$, et le phosphate tétracalcique $Ca_4P_2O_9$ dans des proportions telles que la composition finale soit celle d'une hydroxyapatite de formule :

$$Ca_{10}(PO_4)_6(OH)_2 \quad (II).$$

On utilise avantageusement des mélanges comportant au moins deux composés phosphates, dont un un composé basique (phosphate tétracalcique) et un ou plusieurs composés acides (phosphates dicalcique ou monocalcique).

On peut dans chaque mélange utiliser deux constituants ou plus, ce qui permet de jouer facilement sur la formulation de la céramique apatitique finale.

On peut encore ajouter au mélange de poudres d'autres additifs en vue de conférer à l'hydoxyapatite obtenue des caractéristiques intéressantes pour l'application envisagée.

Ainsi, dans le cas de céramiques apatitiques à usage biologique, il peut être intéressant d'ajouter au mélange de poudres au moins un additif choisi parmi les granulés d'hydroxyapatite préalablement calcinés, des particules de renforcement acceptables du point de vue biologique, des marqueurs radioactifs et les additifs bioactifs dans l'usage orthopédique ou dentaire tels des composés pharmaceutiques, des agents bactéricides, antibiotiques, antibactériens et antimitotiques, et des facteurs de croissance, à condition qu'ils résistent à une température d'au moins 100°C.

Pour des usages non biologiques, d'autres additifs peuvent être utilisés, par exemple des additifs tels que le néodyme en traces et l'europium II ou III en traces, pour leur propriétés de luminescence.

Les granulés d'hydroxyapatites et les particules de renforcement permettent d'améliorer les propriétés mécaniques du matériau. A titre d'exemple de particules de renforcement, on peut citer des particules d'alumine ou de nylon.

L'addition de marqueurs radioactifs peut être intéressante pour suivre la mise en place de l'implant par scintigraphie. A titre d'exemple, ce marqueur radioactif peut être un marqueur au Tc, par exemple du phosphate de technétium-99m.

On peut aussi ajouter aux biomatériaux des composés pharmaceutiques tels que des agents antibiotiques résistant à 100°C au moins, et des agents bactéricides, par exemple de l'argent.

Pour d'autres applications, on peut ajouter des additifs tels que des nylons ou du Kevlar en vue d'améliorer la résistance mécanique.

Après préparation du mélange de poudres comportant éventuellement de tels additifs, on soumet celui-ci à l'étape b) de compactage.

Le compactage est effectué à la température ambiante, par exemple, à une température de 15 à 30°C, sous une pression de 100 à 500 MPa, de préférence de 200 MPa, par exemple au moyen d'une presse hydraulique après avoir disposé le mélange dans un moule.

Dans l'étape suivante c), on soumet la pièce compactée à un traitement hydrothermal dans une enceinte étanche en présence d'un milieu aqueux porté à une température de 100 à 500°C, sous une pression qui correspond à la pression de vapeur d'eau à la température choisie.

De préférence, la pression de vapeur d'eau dans l'enceinte étanche utilisée pour ce traitement est de 0,5 à 17 MPa.

Ce traitement permet d'obtenir une forme céramisée par réaction hydrothermale entre les constituants du mélange compactés. On peut obtenir ainsi des pièces d'une dureté exceptionnelle qui est assurée par le fait que se sont développés à l'intérieur du matériau massif des cristaux aciculaires d'apatite qui conditionnent la cohésion de ce matériau.

Le traitement hydrothermal peut être effectué de deux façons.

Ainsi, selon un premier mode de réalisation du traitement, on immerge totalement la pièce compactée dans le milieu aqueux de façon à ce qu'elle soit en contact avec l'eau à l'état liquide.

Selon un second mode de réalisation de ce traitement hydrothermal, utilisé de préférence pour des pièces compactées renfermant des composés solubles en milieu aqueux, on dispose la pièce compactée au-dessus du milieu liquide de sorte qu'elle n'est en contact qu'avec la vapeur d'eau produite dans l'enceinte étanche, sous l'effet de la température du traitement.

La température du traitement hydrothermal est située dans la gamme de 100 à 500°C, et la durée de ce traitement hydrothermal dépend en particulier de la température utilisée, celle-ci étant plus longue lorsque la température est plus faible. Généralement, la durée est d'au moins 8 heures et elle peut aller jusqu'à 60 heures.

De préférence, la température du traitement hydrothermal est de 150 à 250°C, et sa durée d'environ 48 heures.

Le milieu aqueux utilisé est généralement de l'eau déminéralisée, mais on peut aussi utiliser une solution aqueuse contenant des additifs appropriés.

Ainsi, on peut utiliser, comme milieu aqueux, une solution aqueuse de fluorure de sodium afin d'introduire dans la structure de l'hydroxyapatite des ions fluor, afin de diminuer la solubilité de l'hydroxyapatite. L'addition de fluorure de sodium au milieu aqueux convient en particulier pour la préparation d'une céramique apatitique à usage biologique.

Selon une variante de réalisation du procédé de l'invention, celui-ci comprend de plus une étape complémentaire d) de frittage de la pièce compactée qui a été soumise au traitement hydrothermal. Ce frittage est effectuée à une température d'au moins 1 000°C, par exemple de 1 000 à 1 300°C.

Ainsi, on peut obtenir des matériaux de très haute compacité, possédant d'excellentes propriétés mécaniques.

Le procédé de l'invention est donc très intéressant car il permet d'obtenir diverses compositions de céramiques apatitiques, en fonction des composés utilisés dans le mélange de départ. Par ailleurs, on peut préparer par ce procédé des apatites non-stoechiométriques possédant en particulier un rapport atomique Ca/P inférieur à 1,667 en utilisant dans le mélange des quantités de constituants tels que le rapport global atomique Ca/P soit inférieur à 1,667.

La préparation d'apatites non stoechiométriques présente un grand intérêt car on obtient un composé biocompatible de plus grande solubilité que l'hydroxyapatite stoechiométrique. Cette propriété peut être très intéressante pour la fabrication de pièces susceptibles d'être résorbées et ensuite substituées par un tissu osseux néoformé par l'organisme.

On peut également obtenir une apatite non-stoechiométrique en réglant la durée de l'étape de traitement hydrothermal de façon à obtenir une apatite mélangée à l'un des phosphates de calcium de départ tel que le phosphate tricalcique. En effet, si la durée de réaction est telle que la réaction ne soit pas totale, il peut rester dans la pièce du phosphate tricalcique non transformé en hydroxyapatite par réaction hydrothermale.

D'autres caractéristiques et avantages de l'invention apparaîtront mieux à la lecture des exemples suivants, donnés bien entendu à titre illustratif et non limitatif, en référence au dessin annexé.

**Brève description du dessin.**

La figure 1 est un diagramme de diffraction de rayons X illustrant la structure de la céramique apatitique obtenue dans l'exemple 1.

**Exposé détaillé des modes de réalisation**

**Exemple 1 : Préparation d'hydroxyapatitite phosphocalcique.**

Dans cet exemple, on prépare tout d'abord un mélange de poudres constitué de trois phosphates de calcium qui sont :

- le phosphate monocalcique hydraté, $Ca(H_2PO_4)_2$, $H_2O$,
- le phosphate tricalcique $Ca_3(PO_4)_2$, et
- le phosphate tétracalcique, $Ca_4(PO_4)_2O$.

La proportion de chaque constituant est calculée à partir de la réaction suivante :

$$a\ Ca(H_2PO_4)_2,\ H_2O + b\ Ca_3(PO_4)_2 + c\ Ca_4(PO_4)_2O \xrightarrow{H_2O} Ca_{10}(PO_4)_6(OH)_2.$$

où les coefficients a, b et c sont reliés par la relation suivante :

$$b = 2 - 3a,\ c = 1 + 2a$$

avec $0 \leq a \leq 0,67$ ; $0 \leq b \leq 2$ et $1 \leq c \leq 2,33$.

Dans cet exemple, on utilise les valeurs suivantes :

a = 0,225,
b = 1,325, et
c = 1,45.

Après formation d'un mélange homogène par broyage à une granulométrie inférieure à 100 µm, on dispose le mélange de poudres dans un moule, puis on le soumet à un compactage dans le moule sous une pression de 200 MPa, au moyen d'une presse hydraulique.

Après démoulage de la pièce compactée, on introduit celle-ci dans un autoclave contenant de l'eau déminéralisée de façon qu'elle soit totalement immergée dans l'eau déminéralisée. Après fermeture de l'autoclave, on introduit celui-ci dans une enceinte chauffée où on le porte à une température de 200°C, pendant 48 heures.

On obtient ainsi une pièce en hydroxyapatite calcique très bien cristallisée.

La figure 1 représente le diagramme de diffraction des rayons X du matériau obtenu. On remarque sur cette figure qu'il reste un peu de phosphate tricalcique.

Si l'on augmente le temps de séjour du mélange dans l'autoclave, on fait évoluer en milieu aqueux ce phosphate tricalcique résiduel en hydroxyapatite.

Une étude en microscopie électronique à balayage a montré la présence de gros cristaux aciculaires d'hydroxyapatite calcique, confirmant ainsi la très bonne cristallinité du produit.

La résistance mécanique à la compression de ce matériau est voisine de 100 MPa.

Ce matériau qui est facilement usinable, est particulièrement bien adapté pour des prothèses osseuses.

**Exemple 2 : Préparation d'hydroxyapatites phosphocalciques.**

Dans cet exemple, on prépare un mélange de phosphates comme dans l'exemple 1, puis on le soumet à un compactage dans les mêmes conditions que celles de l'exemple 1.

Après démoulage, on introduit ensuite la pièce compactée dans un autoclave contenant de l'eau déminéralisée, mais on dispose la pièce au-dessus du niveau d'eau afin de réaliser la réaction en milieu de vapeur humide.

Après fermeture de l'autoclave, on introduit celui-ci dans une enceinte chauffée où on le porte à une température de 200°C, pendant 48 heures.

On obtient en fin d'opération un matériau présentant des propriétés analogues à celui obtenu dans l'exemple 1.

**Exemple 3 : Préparation d'une céramique apatitique contenant du strontium.**

Dans cet exemple, on part d'un mélange de poudres de phosphate monocalcique hydraté, de phosphate tétracalcique et de phosphate de strontium et de calcium $Ca_2Sr(PO_4)_2$. La proportion de chaque constituant est calculée à partir de la réaction suivante :

$$a\ Ca(HPO_4)_2, H_2O + b\ Ca_2Sr\ (PO_4)_2 + c\ Ca_4(PO_4)_2O$$

$$\rightarrow Ca_9Sr\ PO_4)_6(OH)_2.$$

avec :

a = 0,34
b = 1
c = 1,66.

On mélange ces constituants par broyage, puis on introduit le mélange de poudres dans une moule et on le comprime au moyen d'une presse hydraulique sous une pression de 190 MPa. On introduit ensuite la pièce comprimée obtenue dans un autoclave et on la recouvre d'eau déminéralisée, puis on ferme l'autoclave et on le porte à une température de 200°C, ce qui correspond à une pression de vapeur d'eau de 1,7 MPa, pendant 72 heures.

On obtient ainsi une hydroxyapatite calcostrontique utilisable comme biomatériau, notamment pour faciliter la repousse osseuse.

Le matériau obtenu présente une résistance à la compression de plus de 100 MPa.

Le procédé de l'invention est particulièrement intéressant car il permet d'obtenir des céramiques apatitiques présentant de bonnes propriétés mécaniques, notamment pour un usage biologique, et d'introduire de plus dans ces

céramiques apatitiques divers additifs pour leur conférer des caractéristiques mécaniques améliorées ou d'autres propriétés.

**Référence citée**

Bioceramics, Vol. 10, 1997, pages 75 à 78.

Tableau :

| **Solubilité des différents phosphates de calcium** | | | |
|---|---|---|---|
| **NOM** | **Formules chimiques** | **Ca/P** | **pKs (à 25°C)** |
| Phosphate Tétracalcique TTCP | $Ca_4(PO_4)_2O$ | 2 | 40 |
| Hydroxyapatite HAp | $Ca_{10}(PO_4)_6(OH)_2$ | 1,667 | 120 |
| Phosphate Tricalcique β β-TCP | $\beta$- $Ca_3(PO_4)_2$ | 1,5 | 28,9 |
| Phosphate Tricalcique α α-TCP | $\alpha$-$Ca_3(PO_4)_2$ | 1,5 | 25,5 |
| Phosphate Octocalcique OCP | $Ca_8H_2(PO4)_65H_2O$ | 1,333 | 96,6 |
| Phosphate Dicalcique dihydraté (Brushite) DCPD | $CaHPO_4,2H_2O$ | 1 | 6,60 |
| Phosphate Dicalcique Anhydre (Monétite) DCPA | $CaHPO_4$ | 1 | 6,90 |
| Phosphate Monocalcique Monohydraté MCPM | $Ca(H_2PO_4)_2$, $H_2O$ | 0,5 | 2,3 |
| Phosphate Monocalcique MCP | $Ca(H_2PO_4)_2$ | 0,5 | 2,4 |

## Revendications

**1.** Procédé de fabrication d'une pièce massive en céramique apatitique, comprenant les étapes suivantes :

a) préparer un mélange homogène de poudres comprenant au moins deux phosphates de calcium choisis parmi : $Ca(H_2PO_4)_2$, $Ca(H_2PO_4)_2.H_2O$, $Ca(HPO_4)$, $Ca(HPO_4).2H_2O$, $Ca_3(PO_4)_2$ de variété α ou β, et $Ca_4(PO_4)_2O$, en quantités telles que le mélange correspond à l'obtention d'une hydroxyapatite stoechiométrique de formule $Ca_{10}(PO_4)_6(OH)_2$ (II) ou non-stoechiométrique de formule :

$$Ca_{10-x}V_x(PO_4)_{6-y}(HPO_4)_y\,(OH)_{2+y-2x} \qquad (III)$$

où V représente une lacune et x et y sont tels que $x < 1$, $y < 1$ et $y \leq x$, ayant un rapport atomique Ca/P inférieur à 1,667 ;

b) compacter le mélange de poudres obtenu dans l'étape a), à la température ambiante, sous une pression de 100 à 500 MPa, pour obtenir une pièce compactée ; et

c) soumettre la pièce compactée à un traitement hydrothermal dans une enceinte étanche contenant un milieu aqueux, à une température de 100 à 500°C, pendant une durée d'au moins 8 heures.

2. Procédé selon la revendication 1, dans lequel, dans l'étape a), on prépare un mélange de poudres comprenant de plus au moins un composé choisi parmi les sels, oxydes et hydroxydes de métaux alcalins, de métaux alcalino-terreux, d'argent ou d'autres métaux, et l'oxyde de silicium, ledit mélange étant capable de former une apatite stoechiométrique de formule :

$$Ca_{10}(PO_4)_6(OH)_2$$

ou une apatite non stoechiométrique de formule :

$$Ca_{10-x}V_x(PO_4)_{6-y}(HPO_4)_y\,(OH)_{2+y-2x} \qquad (III)$$

où V représente une lacune et x et y sont tels que $x < 1$, $y < 1$ et $y \leq x$,
dans lesquelles Ca, $PO_4$ et/ou OH sont remplacés respectivement en partie par d'autres métaux et/ou d'autres anions.

3. Procédé selon la revendication 2, dans lequel les sels sont choisis parmi les phosphates, les silicates, les citrates, les nitrates, les carbonates et les halogénures.

4. Procédé selon la revendication 3, pour la préparation d'une pièce massive en céramique apatitique à usage biologique, dans lequel le mélange de poudres comprend du phosphate de strontium et de calcium de formule :

$$Ca_2Sr(PO_4)_2.$$

ou un composé de strontium de formule : $Sr(H_2PO_4)_2$, $Sr(H_2PO_4)_2.H_2O$, $Sr(HPO_4)$, $Sr(PHO_4).2H_2O$, $Sr_3(PO_4)_2$ et $Sr_4(PO_4)_2O$.

5. Procédé selon la revendication 3 ou 4, pour la préparation d'une pièce massive en céramique apatitique à usage biologique, dans lequel le mélange de poudres comprend du carbonate de calcium.

6. Procédé selon l'une quelconque des revendications 3 à 5, pour la préparation d'une pièce massive en céramique apatitique à usage biologique, dans lequel le mélange de poudres comprend un composé de silicium choisi parmi l'oxyde de silicium $SiO_2$, le métasilicate de calcium $CaSiO_3$ et les métasilicates d'autres métaux.

7. Procédé selon l'une quelconque des revendications 1 à 6, qui comprend en outre une étape complémentaire :

   d) de frittage, à une température d'au moins 1 000°C, de la pièce compactée soumise au traitement hydrothermal, obtenue dans l'étape c).

8. Procédé selon la revendication 1 ou 2, dans lequel, dans l'étape a), on prépare le mélange de poudres par broyage de ces constituants.

9. Procédé selon la revendication 1 ou 2, dans lequel, dans l'étape c), on immerge totalement la pièce compactée dans le milieu aqueux.

10. Procédé selon la revendication 1, dans lequel, dans l'étape c), on dispose la pièce compactée au dessus du milieu aqueux.

11. Procédé selon l'une quelconque des revendications 9 et 10, dans lequel la pression de vapeur d'eau dans l'enceinte étanche utilisée dans l'étape c) est de 0,5 à 17 MPa.

12. Procédé selon l'une quelconque des revendications 1 et 9 à 11, dans lequel la durée du traitement hydrothermal est de 8 à 60 heures.

13. Procédé selon l'une quelconque des revendications 1 et 9 à 12, dans lequel le milieu aqueux est de l'eau déminéralisée.

**14.** Procédé selon l'une quelconque des revendications 1 à 13, pour la préparation d'une pièce massive en céramique apatitique à usage biologique, dans lequel on ajoute au mélange de poudres au moins un additif choisi parmi des granulés d'hydroxyapatite préalablement calcinés, des particules de renforcement acceptables du point de vue biologique, des marqueurs radioactifs, des agents bioactifs choisis parmi les composés pharmaceutiques, les agents bactéricides, antibiotiques, antimitotiques et antibactériens, et les facteurs de croissance.

**15.** Procédé selon la revendication 14, dans lequel le marqueur radioactif est du phosphate de technétium-99m.

**16.** Procédé selon la revendication 14, dans lequel l'agent bactéricide est l'argent.

**17.** Procédé selon l'une quelconque des revendication 1 à 16, pour la préparation d'une pièce massive en céramique apatitique à usage biologique, dans lequel le milieu aqueux utilisé dans l'étape c) comprend du fluorure de sodium.

**18.** Procédé selon l'une quelconque des revendications 1 à 3, dans lequel le mélange de poudres comprend de plus un additif luminescent et/ou un additif pour améliorer la résistance mécanique.

## Claims

**1.** Process for manufacturing a massive apatite ceramic part, including the following steps:

a) preparing a homogeneous mixture of powders including at least two calcium phosphates chosen from: $Ca(H_2PO_4)_2$, $Ca(H_2PO_4)_2 \cdot H_2O$, $Ca(HPO_4)$, $Ca(HPO_4) \cdot 2H_2O$, $Ca_3(PO_4)_2$, $\alpha$ or □ variety, and $Ca_4(PO_4)_2O$, in quantities such that the mixture corresponds to a stoichiometric hydroxyapatite of formula $Ca_{10}(PO_4)_6(OH)_2$ (II) or a non-stoichiometric hydroxyapatite of formula:

$$Ca_{10-x}V_x(PO_4)_{6-y}(HPO_4)_y\,(OH)_{2+y-2x} \qquad (III)$$

in which V represents a flaw and in which x and y are such that $x < 1$, $y < 1$, and $y \leq x$, having a Ca/P atomic ratio less than 1.667.

b) compacting the mixture of powders obtained in step a) at room temperature, under a pressure of 100 to 500 MPa, to yield a compacted piece; and

c) subjecting the compacted piece to hydrothermal treatment in a sealed chamber containing an aqueous medium, at a temperature of 100 to 500°C, for a period of at least 8 hours.

**2.** Process according to claim 1, in which, in step a), a mixture of powders including at least one compound chosen from the salts, oxides and hydroxides of alkaline metals, alkaline-earth metals, silver or other metals, and silicon oxide is prepared, the aforesaid mixture being able to form a stoichiometric apatite of formula:

$$Ca_{10}(PO_4)_6(OH)_2$$

or a non-stoichiometric apatite of formula:

$$Ca_{10-x}V_x(PO_4)_{6-y}(HPO_4)_y\,(OH)_{2+y-2x} \qquad (III)$$

in which V represents a flaw and in which x and y are such that $x < 1$, $y < 1$, and $y \leq x$, in which Ca, $PO_4$ and/or OH are respectively replaced partly by other metals and/or by other anions.

**3.** Process according to claim 2, in which the salts are chosen from among the phosphates, silicates, citrates, nitrates, carbonates and halides.

4. Process according to claim 3, for the preparation of a massive apatite ceramic part for biological use, in which the mixture of powders includes strontium and calcium phosphate of formula:

$$Ca_2Sr(PO_4)_2$$

or a strontium compound of formula: $Sr(H_2PO_4)_2$, $Sr(H_2PO_4)_2 \cdot H_2O$, $Sr(HPO_4) \cdot Sr(HPO_4) \cdot 2H_2O$, $Sr_3(PO_4)_2$ and $Sr_4(PO_4)_2O$.

5. Process according to claim 3 or 4, for the preparation of a massive apatite ceramic part for biological use, in which the mixture of powders includes calcium carbonate.

6. Process according to any of claims 3 to 5, for the preparation of a massive apatite ceramic part for biological use, in which the mixture of powders includes a silicon compound chosen from silicon oxide $SiO_2$, calcium metasilicate $CaSiO_3$, and the metasilicates of other metals.

7. Process according to any of claims 1 to 6, which also includes an additional step:

d) of sintering at a temperature of at least 1000°C, of the compacted piece subjected to hydrothermal treatment obtained in step c).

8. Process according to claim 1 or 2, in which, in step a), the mixture of powders is prepared by grinding the ingredients.

9. Process according to claim 1 or 2, in which, in step c), the compacted piece is totally immersed in the aqueous medium.

10. Process according to claim 1, in which, in step c), the compacted piece is arranged above the aqueous medium.

11. Process according to any of claims 9 and 10, in which the water vapour pressure in the sealed chamber used in step c) is from 0.5 to 17 MPa.

12. Process according to any of claims 1 and 9 to 11, in which the duration of hydrothermal treatment is from 8 to 60 hours.

13. Process according to any of claims 1 and 9 to 12, in which the aqueous medium is demineralized water.

14. Process according to any of claims 1 to 13, for the preparation of a massive apatite ceramic part for biological use, in which at least one additive chosen from granulates of previously calcinated hydroxyapatite, reinforcement particles which are acceptable from a biological standpoint, radioactive tracers, bioactive agents chosen from pharmaceutical compounds, bactericide, antibiotic, antibacteria and antimitotic agents, and growth factors, is added to the powder mixture.

15. Process according to claim 14, in which the radioactive tracer is Technetium-99m Phosphate.

16. Process according to claim 14, in which the bactericide agent is silver.

17. Process according to any of claims 1 to 16, for the preparation of a massive apatite ceramic part for biological use, in which the aqueous medium used in step (c) includes sodium fluoride.

18. Process according to any of claims 1 to 3, in which the mixture of powders includes a luminous additive and/or an additive to improve mechanical resistance.

**Patentansprüche**

1. Verfahren zur Herstellung eines massiven Stücks aus Apatitkeramik, die folgenden Schritte umfassend:

a) Herstellen einer homogenen Pulvermischung, wenigstens zwei unter: $Ca(H_2PO_4)_2$, $Ca(H_2PO_4)_2 \cdot H_2O$, Ca

$(HPO_4)$, $Ca(HPO_4) \cdot 2H_2O$, $Ca_3(PO_4)_2$ von α- oder β-Varietät und $Ca_4(PO_4)_2O$ gewählte Calciumphosphate in solchen Mengen umfassend, dass die Mischung der Gewinnung eines stöchiometrischen Hydroxyapatits der Formel $Ca_{10}(PO_4)_6(OH)_2$ (II) oder eines ein Atomverhältnis Ca/P unter 1,667 besitzenden nichtstöchiometrischen Hydroxyapatits der Formel:

$$Ca_{10-x}V_x(PO_4)_{6-y}(HPO_4)_y(OH)_{2+y-2x} \quad (III)$$

entspricht, wo V eine Lücke darstellt und x und y derart sind, dass $x < 1$, $y < 1$ und $y \leq x$;
b) Verdichten der im Schritt a) erhaltenen Pulvermischung bei Umgebungstemperatur unter einem Druck von 100 bis 500 MPa, um ein verdichtetes Stück zu erhalten; und
c) Unterziehen des verdichteten Stücks einer hydrothermalen Behandlung in einem dichten Behälter, der ein wässriges Medium enthält, bei einer Temperatur von 100 bis 500°C während einer Dauer von wenigstens 8 Stunden.

**2.** Verfahren nach Anspruch 1, in welchem man im Schritt a) eine Pulvermischung herstellt, die darüber hinaus wenigstens eine unter den Salzen, Oxiden und Hydroxiden von Alkalimetallen, Erdalkalimetallen, Silber oder anderen Metallen gewählte Verbindung und Siliciumoxid umfasst, wobei die genannte Mischung fähig ist, einen stöchiometrischen Apatit der Formel:

$$Ca_{10}(PO_4)_6(OH)_2$$

oder einen nichtstöchiometrischen Apatit der Formel:

$$Ca_{10-x}V_x(PO_4)_{6-y}(HPO_4)_y(OH)_{2+y-2x} \quad (III)$$

wo V eine Lücke darstellt und x und y derart sind, dass $x < 1$, $y < 1$ und $y \leq x$, zu bilden,
in welchen Ca, $PO_4$ und/oder OH jeweils zum Teil durch andere Metalle und/oder andere Anionen ersetzt sind.

**3.** Verfahren nach Anspruch 2, in welchem die Salze unter den Phosphaten, den Silicaten, den Citraten, den Nitraten, den Carbonaten und den Kalogeniden gewählt sind.

**4.** Verfahren nach Anspruch 3 für die Herstellung eines massiven Stücks aus Apatitkeramik zum biologischen Gebrauch, in welchem die Pulvermischung Strontium-Calcium-Phosphat der Formel:

$$Ca_2Sr(PO_4)_2$$

oder eine Strontiumverbindung der Formel: $Sr(H_2PO_4)_2$, $Sr(H_2PO_4)_2 \cdot H_2O$, $Sr(HPO_4)$, $Sr(HPO_4) \cdot 2H_2O$, $Sr_3(PO_4)_2$ und $Sr_4(PO_4)_2O$ umfasst.

**5.** Verfahren nach Anspruch 3 oder 4 für die Herstellung eines massiven Stücks aus Apatitkeramik zum biologischen Gebrauch, in welchem die Pulvermischung Calciumcarbonat umfasst.

**6.** Verfahren nach einem der Ansprüche 3 bis 5 für die Herstellung eines massiven Stücks aus Apatitkeramik zum biologischen Gebrauch, in welchem die Pulvermischung eine Siliciumverbindung umfasst, die unter Siliciumoxid $SiO_2$, Calciummetasilicat $CaSiO_3$ und den Metasilicaten anderer Metalle gewählt ist.

**7.** Verfahren nach einem der Ansprüche 1 bis 6, das ferner einen ergänzenden Schritt:

d) der Sinterung des in Schritt c) erhaltenen verdichteten, der hydrothermalen Behandlung unterworfenen Stücks bei einer Temperatur von wenigstens 1000°C umfasst.

**8.** Verfahren nach Anspruch 1 oder 2, in welchem man im Schritt a) die Pulvermischung durch Vermahlen ihrer Bestandteile herstellt.

**9.** Verfahren nach Anspruch 1 oder 2, in welchem man im Schritt c) das verdichtete Stücke vollständig in das wässrige Medium eintaucht.

**10.** Verfahren nach Anspruch 1, in welchem man in Schritt c) das verdichtete Stück über dem wässrigen Medium anordnet.

**11.** Verfahren nach einem der Ansprüche 9 und 10, in welchem der Druck von Wasserdampf in dem im Schritt c) verwendeten dichten Behälter 0,5 bis 17 MPa beträgt.

**12.** Verfahren nach einem der Ansprüche 1 und 9 bis 11, in welchem die Dauer der hydrothermalen Behandlung 8 bis 60 Stunden beträgt.

**13.** Verfahren nach einem der Ansprüche 1 und 9 bis 12, in welchem das wässrige Medium demineralisiertes Wasser ist.

**14.** Verfahren nach einem der Ansprüche 1 bis 13 für die Herstellung eines massiven Stücks aus Apatitkeramik zum biologischen Gebrauch, in welchem man der Pulvermischung wenigstens einen unter vorher calcinierten Hydroxyapatitkörnern, vom biologischen Gesichtspunkt annehmbaren Verstärkungspartikeln, radioaktiven Markem, Mitteln, die unter den pharmazeutischen Verbindungen, den bakteriziden, antibiotischen, antimitotischen und antibakteriellen Mitteln und den Wachstumsfaktoren gewählt sind, gewählten Zusatzstoff hinzufügt.

**15.** Verfahren nach Anspruch 14, in welchem der radioaktive Marker Technetium-99m-phosphat ist.

**16.** Verfahren nach Anspruch 14, in welchem das bakterizide Mittel Silber ist.

**17.** Verfahren nach einem der Ansprüche 1 bis 16 für die Herstellung eines massiven Stücks aus Apatitkeramik zum biologischen Gebrauch, in welchem das in Schritt c) verwendete wässrige Medium Natriumfluorid umfasst.

**18.** Verfahren nach einem der Ansprüche 1 bis 3, in welchem die Pulvermischung darüber hinaus einen lumineszierenden Zusatzstoff und/oder einen Zusatzstoff, um die mechanische Beständigkeit zu verbessern, umfasst.

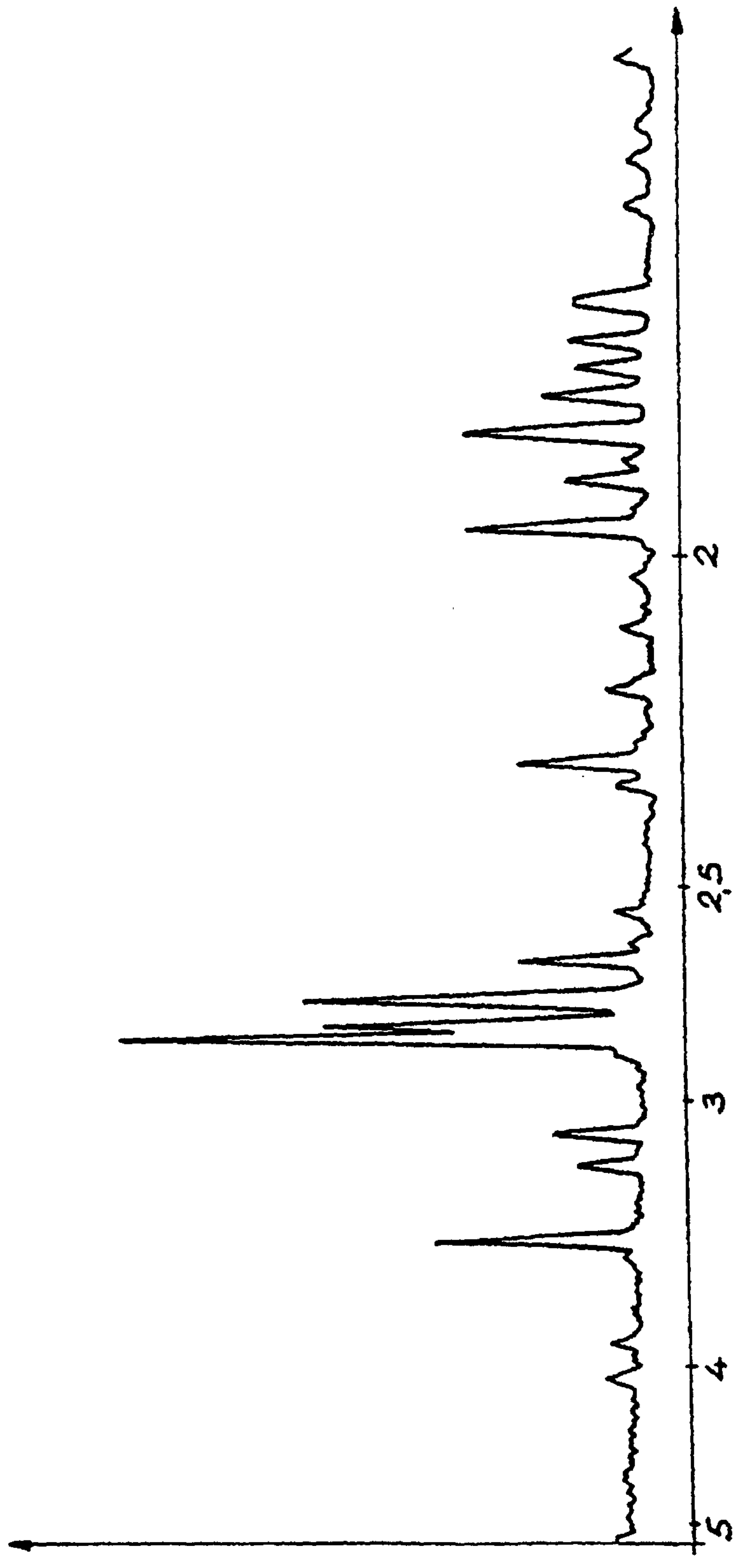
2
2,5
3
4
5